## Europäisches Patentamt

## European Patent Office

## Office européen des brevets

(19)

(11) Veröffentlichungsnummer: **0 272 312 B1**

(12) **EUROPÄISCHE PATENTSCHRIFT**

(45) Veröffentlichungstag der Patentschrift: **23.01.91**

(51) Int. Cl.⁵: **A 23 L 2/40, A 61 K 9/46**

(21) Anmeldenummer: **87904519.3**

(22) Anmeldetag: **24.06.87**

(86) Internationale Anmeldenummer: **PCT/EP87/00331**

(87) Internationale Veröffentlichungsnummer: **WO 88/00009 14.01.88 Gazette 88/02**

(54) **VERFAHREN ZUM HERSTELLEN EINES BRAUSEGRANULATES, DANACH HERGESTELLTES BRAUSEGRANULAT SOWIE DESSEN VERWENDUNG.**

(30) Priorität: **26.06.86 DE 3621432**
**13.08.86 DE 3627475**

(43) Veröffentlichungstag der Anmeldung:
**29.06.88 Patentblatt 88/26**

(45) Bekanntmachung des Hinweises auf die Patenterteilung:
**23.01.91 Patentblatt 91/04**

(84) Benannte Vertragsstaaten:
**AT BE CH DE FR GB IT LI LU NL SE**

(56) Entgegenhaltungen:
**AT-A- 374 348**
**DE-A-3 149 517**
**DE-A-3 434 774**
**US-A-3 102 075**
**US-A-3 359 119**
**US-A-3 401 216**

(73) Patentinhaber: **Gergely, Gerhard, Dr.**
**Gartengasse 8**
**A-1050 Wien (AT)**

(72) Erfinder: **GERGELY, Irmgard**
**Gartengasse 8**
**A-1050 Wien (AT)**
Erfinder: **GERGELY, Stefan, M.**
**Gartengasse 8**
**A-1050 Wien (AT)**
Erfinder: **GERGELY, Thomas**
**Gartengasse 8**
**A-1050 Wien (AT)**
Erfinder: **GERGELY, Gerhard**
**Gartengasse 8**
**A-1050 Wien (AT)**

(74) Vertreter: **Goddar, Heinz J., Dr. et al**
**FORRESTER & BOEHMERT Widenmayerstrasse 4/I**
**D-8000 München 22 (DE)**

Courier Press, Leamington Spa, England.

**Beschreibung**

Die Erfindung betrifft ein Verfahren zum Herstellen eines Brausegranulates mit einem Gehalt an wenigstens einer festen, kristallinen, eßbaren organischen Säure, insbesondere Zitronensäure, und mindestens einem bei Reaktion mit der organischen Säure in wäßriger Lösung $CO_2$ abspaltenden Alkali- oder Erdalkalimetallcarbonat, ein danach hergestelltes Brausegranulat sowie dessen Verwendung.

Aus der DE—OS 34 34 774 sind ein Brausegranulat der vorstehend beschriebenen Art sowie ein Verfahren zu seiner Herstellung bekannt, bei denen organische Säurekristalle, insbesondere Zitronensäurekristalle, durch Anreagieren im Vakuum mit einem mehrschichtigen Calciumcarbonat-haltigen Überzug versehen werden. Dabei wird so vorgegangen, daß in einer Vakuummischmaschine die Säurekristalle in einem ersten Anreaktionsschritt mit einem Lösungsmittel für die Säure, wie Wasser, Alkohol oder einer Mischung hieraus, benetzt und alsdann zur Ausbildung einer ersten über eine durch Reaktion von Säure und Carbonat gebildete Bindeschicht an der Oberfläche des jeweiligen Säurekristalls haftenden Überzugsschicht mit einer ersten pulverisierten Überzugsmasse mit einen Gehalt an mindestens einem Alkali- oder Erdalkalimetallcarbonat intensiv durchmischt werden, woraufhin nach Bildung der ersten Überzugsschicht in mindestens einem weiteren Anreaktionsschritt mindestens eine weitere, ggf. mit der ersten übereinstimmende feste pulverisierte Übersuzmasse mit einem Gehalt an mindestens einem Alkali- oder Erdalkalimetallcarbonat zugesetzt und an der im vorausgehenden Anreaktionsschritt gebildeten, durch das bei der Anreaktion abgespaltene kristallwasserfeuchten vorangehenden Überzugsschicht zur Ausbildung mindestens einer weiteren Überzugsschicht unter intensivem Rühren anreagiert wird, woraufhin schließlich nach Abbrechen der Überzugsbildung eine Endtrocknung erfolgt; die vorgenannte Vorgehensweise nach der DE—OS 34 34 774 wird im folgenden u.a. auch als "gattungsgemäßes Verfahren" bezeichnet.

Das vorstehend beschriebene Brausegranulat sowie das gattungsgemäße Verfahren zu seiner Herstellung haben sich in der Praxis hervorragend bewährt, wobei es sich aber als wünschenswert herausgestellt hat, das zu seiner Herstellung angewendete Verfahren, welches beim gattungsgemäßen Stand der Tecknik in einer Vakuummischmaschine durchgeführt wird, noch dahingehend weiter zu verbessern, daß ggf. auch ohne Verwendung einer Vakuummischmaschine Brausegranulate in der gewünschten Qualität hergestellt werden können, sich aber auch bei Anwendung des bekannten Vakuummischverfahrens Vorteile hinsichtlich einer rascheren Verfahrensführung sowie in der Stabilität der hergestellten Brausegranulate ergeben, insbesondere dann, wenn letztere besonders reaktionsfähige Alkali-bzw. Erdalkalimetallcarbonate sind oder aber diese in sehr hoher Konzentration enthalten, da es in diesen Fällen bei bestimmten Konditionen bei Anwendung des bekannten Verfahrens der gattungsgemäß vorausgesetzten Art wegen allzu heftiger Reaktionen in der Vakuummischmaschine zu Schwierigkeiten kommen kann. Wird dabei nämlich zuviel Carbonat mit der organischen Säure in der Vakuummischmaschine in einer bestimmten Verfahrensstufe zur Reaktion gebracht und/oder zuviel Lösung verwendet, so erfolgt eine unerwünschte, allzu große Agglomeration im Inneren des Kessels der Vakuummischmaschine, wodurch eine gewisse Klumpenbildung resultiert und auch der Rührer im Kessel blockiert werden kann, so daß sich das erwünschte Ziel einer zuverlässigen Ummantelung der Säurekristallpartikel mit den carbonathaltigen Schichten nicht erreichen läßt.

Der Erfindung liegt daher die Aufgabe zugrunde, das gattungsgemäße Verfahren dahingehend zu verbessern, daß auch ohne Verwendung einer Vakuummischmaschine Brausegranulate hoher Qualität und Stabilität hergestellt werden und insgesamt eine raschere Verfahrensführung auch bei heftig miteinander reagierenden Substanzkombinationen für die Säurekristalle einerseits und die Überzugsschichten andererseits gewährleistet werden kann.

Erfindungsgemäß wird diese Aufgabe in Weiterbildung des gattungsgemäßen Verfahrens durch die im Patentanspruch 1 aufgeführten Merkmale gelöst.

Besonders bevorzugte Ausführungsformen des Verfahrens nach der Erfindung sind Gegenstand der Patentansprüche 2 bis 15. Das Brausegranulat nach der Erfindung ist Gegenstand des Patentanspruches 16, während dessen Verwendung Gegenstand der Patentansprüche 17, 18 und 19 ist.

Der Erfindung liegt die überraschende Erkenntnis zugrunde, daß es gelingt, die wünschenswerte Anreaktion ein- oder mehrfacher alkali- oder erdalkalimetallcarbonathaltiger Überzugsschichten z.B. an Zitronensäurekristalle ggf. auch ohne Verwendung eines Vakuummischverfahrens, wie es z.B. in der AT—PS 37 61 47 beschrieben ist, zu bewerkstelligen, wenn vor dem Aufbringen auf die Säurekristalle ein Teil der z.B. Zitronensäure sowie des z.B. Calciumcarbonates miteinander in einem entsprechenden Lösungsmittel, also in Wasser, Alkohol oder in einem Alkohol-Wasser-Gemisch, zur Reaktion gebracht werden und die so hergestellte Vorreaktionslösung dann auf die Säurekristalle aufgebracht wird. Dies stellt eine unerwartet vorteilhafte Weiterbildung der z.B. aus der DE—OS 34 34 774 vorbekannten Vorgehensweise dar, wonach man auf der Oberfläche der z.B. Zitronensäurekristalle mit mehr oder weniger polaren Lösungsmitteln Anreaktionen mit Alkali- oder Erdalkali-Bicarbonaten oder auch -carbonaten durchführen kann und die dabei entstehenden Reaktionsprodukte, die im vorgenannten Fall also im wesentlichen aus Mono- oder ggf. Di-Salzen der Zitronensäure bestehen, als Bindemittel für die jeweiligen nächsten Schichten oder aber für den Einbau von Salzen oder dergleichen dienen. Die Problematik der bisher bekannten Vorgehensweise läßt sich auch bei Verwendung von Vakuummischverfahren durch die erfindungsgemäße Lehre vollkommen vermeiden, indem nämlich dabei nicht mehr die Schwierigkeit auftreten kann, daß bei zu heftiger Reaktion, also bei Einbringen von zuviel Flüssigkeit in die Vakuummischmaschine, eine unerwünschte, zu große Agglomeration im Inneren des Kessels derselben erfolgt, die dan zu Klumpenbildung führt und auch das Ergebnis haben kann, daß der Rührere im Kessel blockiert und das gewünschte Ziel einer Ummantelung der Säurekristalle nicht vollständig erreicht wird. Erfindungsgemäß wird daher zumindest ein Teil dieser Reaktion außerhalb des Kessels durchgeführt, indem z.B. sowohl bei Calciumprodukten als auch bei Kaliumprodukten Zitronensäure mit Calciumcarbonat oder Kaliumbicarbonat in derart konzentrierter Form zur Reaktion gebracht wird, daß die endkonzentrierte Lösung blank ist und zumindest auf einige Zeit keine Kristallisation zeigt. Diese sehr konzentriert Vorreaktionslösung wird dann in die Vakuummischmaschine eingesaugt und hat den Vorteil, dort mit den darin befindlichen Säurekristallen keine oder nur mehr eine sehr geringe Reaktion auszulösen, je nachdem, ob man ein Mono-Calciumcitrat oder ein Di-Calciumcitrat, beim vorgenannten Beispiel, in Lösung herstellt. Die Herstellung einer Tri-Calciumcitrat-Lösung ist ja bekanntlich nicht möglich, weil dieses wasserunlöslich ist. Saugt man nun auch Zitronensäure schon im Beginn eines ersten Anreaktionsschrittes oder auch in weiteren Anreaktionsschritten oder -stufen statt mit einem Lösungsmittel allein als eine bereits vorreagierte Lösung, nämlich die erfindungsgemäß verwendete Vorreaktionslösung, der Reaktionspartner ein, so ist diese hervorragend geeignet, als Bindemittel für weitere Calciumcarbonat-, Kaliumbicarbonat- oder auch Natriumbicarbonat-Überzugsschichten zu dienen. Nach Herstellung der entsprechenden Überzugsschichten kann es von Vorteil sein, relativ große Mengen an Alkohol, am besten Isopropanol, zuzusetzen. Diese Alkahole fällen dann die anorganischen Salze der organischen Säure, insbesondere der Zitronensäure, aus, und zwar selbstverständlich in wasserfreier Form, da in Anwesenheit von Alkohol eine Kristallisation unter einem Einbau von Kristallwasser nicht möglich ist. Bevor dieser Alkohol verdampft, kann man beispielsweise Stärke oder auch Neutralsalze, wie Calciumlactat oder dergleichen, einbringen und diese Salze gleichsam "aufkleben", woraufhin dann erst das gesamte vorliegende Wasser mit dem Alkohol als Trägermittel entfernt wird. Dieser Vorgang ist besonders vorteilhaft, weil die überaus Konzentrierten Lösungen von insbesondere Kaliumcitraten nur einen sehr geringen Dampfdruck zeigen, d.h. also, eine derartige Lösung würde nicht mehr bei 100°C sieden, sondern bei 150°C oder gar erst bei 180°C, so daß eine Verdampfung im Vakuum bei beispielsweise 70°C nicht ohne weiteres möglich ist. Wird aber in der erfindungsgemäß vorgeschlagenen Weise unter Zugabe von Alkohol ein Ausfällen der Salze bewirkt und löst man dabei das vorhandene Wasser gleichzeitig im Alkohol, dann treten wieder normale Dampfdruckverhältnisse ein, so daß sich mit reduziertem Druck, bei Anwendung des Vakuummischverfahrens, sowohl das Wasser als auch der Alkohol oder aber beide zusammen (azeotrop bei Isopropanol) zur Verdampfung bringen lassen. Auch dieser Vorgang läßt sich ggf. mehrfach wiederholen.

Die besonders bevorzugte Ausführungsform der Erfindung, bei der mindestens einer calciumcarbonathaltigen Überzugsschicht einerseits wasserfreie Stärke und zum anderen wasserfreie Calciumsalze der beanspruchten Art zugesetzt werden, hat den besonderen Vorteil, daß sich die Calciumkonzentration des Brausegranulates ohne Beeinträchtigung der Auflösungsgeschwindigkeit, ja unter drastischer Steigerung derselben bei weiterer deutlicher Verbesserung der Haltbarkeit des Brausegranulates erhöhen läßt. Dadurch, daß diese Additive wasserfrei verwendet werden, wirken sie selbst als zusätzliches Trocknungsmittel beim Lagern des Granulates bzw. einer Brausetablette, indem sie verhindern, daß zwischen dem Calciumcarbonat und der Zitronensäure eine Wasser freisetzende Kettenreaktion in Gang gesetzt wird, die zu einem vorzeitigen Altern und Unbrauchbarwerden des Brausegranulates bzw. der Brausetablette führen würden. Ferner wirkt die Stärke dabei als Sprengmittel für die ansonsten schwerlöslichen und/oder mit der eßbaren organischen Säure nicht reaktionsfähigen zusätzlichen Calciumsalze, so daß diese beim Auflösen des Brausegranulates besonders rasch unter Ausnutzung der mechanischen Sprengwirkung im Wasser verteilt werden. Erfindungsgemäß bei dieser bevorzugten Ausführungsform hergestellte Brausegranulate eignen sich bevorzugt insbesondere als Instant-Präparat, wobei die Auflösungszeit um ca. 20 bis 30 Sekunden bei ca. 5°C beträgt, verglichen mit dem Calciumcarbonatpräparat, welches bei derartig niedrigen Temperaturen untolerierbare, mehrere Minuten lange Auflösungszeiten erfordert.

Für die Erfindung ist es besonders wichtig, daß die Stärke in völlig wasserfreiem Zustand verwendet wird, da nur dann eine entsprechende quasimechanische Sprengwirkung gewährleistet wird, die die vorstehend beschriebenen kurzen Auflösungszeiten garantiert. Nach dem erfindungsgemäßen Verfahren hergestellte Brausegranulate sind so haltbar, daß sie wegen der synergistisch erzielten "Trockenmitteleigenschaften" der wasserfreien Stärke einerseits und der schwerlöslichen bzw. mit der eßbaren organischen Säure nicht reaktionsfähigen wasserfreien Calciumsalze andererseits praktisch in offener Flasche aufbewahrt werden können.

Festzuhalten ist in jedem Fall, daß der erfindungsgemäße Gedanke der Verwendung vorreagierter "Puffergranulierungslösungen" sich zwar vorzugsweise beim Vakuummischverfahren der gattungsgemäß vorausgesetzten Art eignet, jedoch auch bei einer "Freiluftgranulierung", also einer Granulierung ohne Verwendung von Vakuummischmaschinen, zu den beschriebenen erheblichen Vorteilen führt, wobei dann also eine Granulierung z.B. in Mischwerken oder dergleichen im großtechnischen Maßstab erfolgen und die Endtrocknung über Bandtrockner oder dergleichen durchgeführt werden kann.

Erfindungsgemäß lassen sich auch, wie beansprucht, isotonische und hypertonische Brausegranulate herstellen, die sich dadurch auszeichnen, daß sie bis zu einem Verhältnis von etwa 1:5 mit Instantzucker gemischt werden können und infolge der Dichtheit des Granulates und dessen überraschend hoher lokaler Brausefähigkeit in der Lage sind, bis zur 5-fachen Menge Instantzucker durchzuwirbeln und gleichzeitig zur Auflösung zu bringen. Dadurch lassen sich Instant-Softdrinks herstellen, die wie die üblichen Softdrinks 8 bis 10 Gew.% Zucker enthalten, jedoch auch die notwendige Menge an Zitronensäure, Mineralsalzen, wie Calcium, Magnesium, Natrium und Kalium, selbstverständlich mit Aromazusätzen. Derartige Instantprodukte unterscheiden sich in äußerst vorteilhafter Weise von den bislang bekannten ähnlichen Instantprodukten, die sich im Wasser nur sehr langsam unter Rühren auflösen und außerdem relativ flau schmerkken.

Beispiel 1

Besonders vorteilhaft ist die erfindungsgemäße Vorgehensweise bei der Handhabung hygroskopisch schwieriger Kaliumsalze. Hierzu werden 250 Gewichtsteile kristallesierter Zitronensäure mit einer Korngröße von 0,4 bis 0,6 mm mit einer Lösung von 20 Gewichtsteilen Zitronensäure in 15 Gewichtsteilen Wasser, mit Zusatz von 20 Gewichtsteilen Kaliumbicarbonat, versetzt. Diese Lösung wird in einem hochtourigen Rührwerk unter Anwärmen auf 35°C gerührt und so eine Vorreaktionslösung hergestellt. Anschliessend werden 210 Gewichtsteile pulverisierten verisierten Kaliumbicarbonates zugefügt. Die praktisch aus Di-Kaliumcitrat bestehende Vorreaktionslösung wirkt dabei als Bindemittel und Puffer zwischen der Zitronensäure und dem Kaliumbicarbonat, wobei eine heftige Reaktion nicht auftreten kann, da Di-Kalliumcitrat bereits sehr langsam und träge mit Kaliumbicarbonat reagiert. Hierdurch läßt sich nicht nur eine Granulierung, d.h. ein Aufbau eines gleichmäßig rieselfähigen Gebildes erzielen, vielmehr ist auch gewährleistet, daß zwischen den Zitronensäurekristallen einerseits und dem sehr stark hygroskopischen Kaliumbicarbonat andererseits eine Passivierungsschicht eingebaut wird. Bei dieser Vorgehensweise ist es ratsam und vorteilhaft, bei Beendigen von ein oder zwei Überzugsvorgängen der vorstehend beschriebenen Art eine größere Menge Alkohol, vorzugsweise das 3- bis 4-fache der vorher eingesetzten Wassermenge, zuzufügen, um die alkoholunlöslichen Kaliumsalze wasserfrei auszufällen und das als Lösungsmittel dienende Wasser mit Alkohol, im Falle von Isopropanol in besonders vorteilhafter Weise azeotrop, zu entfernen.

Durch diese Vorgehensweise werden hervorragend rieselfähige Granulate geschaffen, die sich zu äußerst harten Brausetabletten verpressen lassen, wobei diese Brausetabletten eine sehr stark reduzierte Empfindlichkeit gegen Luftfeuchtigkeit haben, da auch Di-Kaliumcitrat in der Lage ist, Kristallwasser einzubauen, im Gegensatz zu Mono-Kaliumcitrat. Eine ähnliche Möglichkeit besteht bei Tri-Kaliumcitrat, wobei die Vorreaktionslösung dann nicht, wie vorstehend beschrieben, 20 Gewichtsteile Kaliumcarbonat, sondern 30 Gewichtsteile Kaliumbicarbonat enthält. In diesem Falle granuliert man also mit einer Lösung von Tri-Kaliumcitrat, die ebenfalls wiederum durch Alkohol wasserfrei ausgefällt wird und eine Passivierungsschicht zwischen den einzelnen Partnern, d.h. Säurekristallen und der aus der ersten Überzugsschicht bzw. zwei aufeinander folgenden Überzugsschichten, bildet.

Erfahrungsgemäß hat sich allerdings Di-Kaliumcitrat besser bewährt, weil die Klebekraft dieses Salzes, welches dann später wahrscheinlich ein Gemisch von Mono-, Di- und Tri-Kaliumcitraten im Rahmen der Reaktion darstellt, wesentlich bessere Bindungseigenschaften gewährleistet als auch ein definiertes Tri-Kaliumcitrat, um nur ein Neispiel zu nennen.

Beispiel 2

Es wird vorgegangen wie in Beispiel 1, wobei aber zusätzlich eine Überzugsschicht aus Magnesiumoxid aufgebracht wird. Hierzu werden 90 Gewichtsteile kristallisierter Zitronensäure mit der Di-Kaliumcitrat-Lösung versetzt. Die wie in Beispiel 1 hergestellte Vorreaktionslösung wird dann durch Mischen auf den Zitronensäurekristallen verteilt. Danach werden 20 Gewichtsteile Kaliumcarbonat aufgebracht. Alsdann wird weitere Vorreaktionslösung eingebracht, woraufhin dann 11 Gewichtsteile Magnesiumoxid als Zwischenschicht verankert werden. Abschließend werden dann weitere Vorreaktionslösung sowie 25 Gewichtsteile Kaliumcarbonat aufgebracht, um so eine abschließende Überzugsschicht zu erhalten, ehe dann unter Rühren endgetrocknet wird.

Insgesamt läßt sich mittels der erfindungsgemäßen Vorgehensweise die Notwendigkeit vermeiden, auch dann, wenn kein Vakuummischverfahren angewendet wird, also eine Granulierung unter atmosphärischen Bedingungen stattfindet, nach dem nassen Verpressen der noch feuchten Granulate eine längere Hitzebehandlung der Brausetabletten, aber auch der Brausegranulate, durchführen zu müssen. Während einer derartigen Hitzebehandlung, wie sie beim Stand der Technik üblich ist, verdampft das Wasser teilweise, wobei Reaktionen der vorstehend beschriebenen Art, wie sie erfindungsgemäß im Vakuum durchgeführt werden im Endprodukt erfolgen, selbstverständlich ohne daß dabei noch die Entfernung von Kristallwasser möglich wäre.

Auch werden bei der Vorgehensweise bei dem Stand der Technik beigefügte Inhaltsstoffe, insbesondere Vitamine, aber auch Aromastoffe, durch die Feuchtigkeit und die sauren bzw. stark alkalisch Reaktionspartner zerstört, so daß eine derartige Vorgehensweise für empfindliche Wirkstoffe nicht geeignet ist. Auch insofern ergibt sich demzufolge ein erheblicher Vorteil der erfindungsgemäßen Vorgehensweise gegenüber dem als "curing" insbesondere in der US-amerikanischen Literatur beschriebenen Prozeß, nämlich der Behandlung von naßgepressten Tabletten durch anschließende Endtrocknung mittels längerer Hitzebehandlung (z.B. Herbert A. Liebermann und Leon Lachmann "Pharmaceutical dosage forms — tablets", Vol. 1, Seiten 232—243).

## Patentansprüche

1. Verfahren zum Herstellen eines Brausegranulates mit einem Gehalt an wenigstens einer festen, kristallinen, eßbaren organischen Säure und mindestens einem bei Reaktion mit der organischen Säure in wäßriger Lösung $CO_2$ abspaltenden Alkali- oder Erdalkalimetallcarbonat, gekennzeichnet durch die folgenden Schritte:

(a) Ein Teil der gesamten Säuremenge und ein Teil des/der Alkalimetallcarbonate(s) einer ersten pulverisierten Überzugsmasse, die einen Gehalt an mindestens einem Alkali- oder Erdalkalimetallcarbonat aufweist, werden in einem Lösungsmittel für die Säure gelöst und miteinander zur Reaktion gebracht;

(b) Die nicht im Verfahrensschritt (a) zur Herstellung der darin gebildeten Vorreaktionslösung verwendeten Säurekristalle werden mit der im Verfahrensschritt (a) hergestellten Vorreaktionslösung benetzt;

(c) Die mit der Vorreaktionslösung im Schritt (b) benetzten Säurekristalle werden mit dem nicht für die Herstellung der Vorreaktionslösung verwendeten Teil der ersten pulverisierten Überzugsmasse intensiv durchmischt, wodurch eine erste Überzugsschicht an den Säurekristallen gebildet wird, die über eine durch Reaktion von Säure und Carbonat gebildete Bindeschicht an der Oberfläche der Säurekristalle haftet;

(d) Nach Bildung der ersten Überzugsschicht wird mindestens eine weitere feste pulverisierte Überzugsmasse mit einem Gehalt an mindestens einem Alkali- oder Erdalkalimetallcarbonat den im Schritt (c) mit der ersten Überzugsschicht versehenen Säurekristallen unter intensivem Rühren zugesetzt, wodurch sich an der vorausgehenden Überzugsschicht, welche durch abgespaltenes Kristallwasser feucht ist, mindestens eine weitere Überzugsschicht bildet;

(e) Nach Abbrechen der Überzugsbildung erfolgt eine Endtrocknung.

2. Verfahren nach Anspruch 1, dadurch gekennzeichnet, daß nach Bildung der ersten Überzugsschicht und/oder mindestens einer der nachfolgenden Überzugsschichten eine Zwischenbenetzung mit einer durch Vorreaktion eines (jeweils) weiteren Teiles der gesamten Säuremenge und eines Teiles des/der Alkali- und/oder Erdalkalimetallcarbonate(s) der nächstfolgenden Überzugsmasse in weiterem Lösungsmittel gebildeten jeweils weiteren Vorreaktionslösung erfolgt.

3. Verfahren nach Anspruch 1 oder 2, dadurch gekennzeichnet, daß das Lösungsmittel vor dem Einbringen der Säure und/oder des/der Alkali- oder Erdalkalimetallcarbonate(s) auf etwa 30 bis 40°C angewärmt wird.

4. Verfahren nach einem der vorangehenden Ansprüche, dadurch gekennzeichnet, daß die Vorreaktionslösung(en) vor dem Aufbringen auf die Säurekristalle auf etwa 30 bis 40°C angewärmt wird/werden.

5. Verfahren nach einem der vorangehenden Ansprüche, dadurch gekennzeichnet, daß die Vorreaktionslösung(en) in derartiger Konzentration hergestellt wird/werden, daß sie gerade noch blank ist/sind und in dem bei der Verfahrensdurchführung erforderlichen Zeitraum keine sichtbare Auskristallisation erfolgt.

6. Verfahren nach einem der vorangehenden Ansprüche, dadurch gekennzeichnet, daß die feste(n) pulverförmigen(n) Überzugsmasse(n) (jeweils) einen Teil der gesamten Säuremenge enthält/enthalten.

7. Verfahren nach einem der vorangehenden Ansprüche, dadurch gekennzeichnet daß nach Aufbringen mindestens einer, ggf. der letzten Überzugsschicht ein mindestens einmaliges Aufbringen von Alkohol mit anschließenden Trocknen erfolgt.

8. Verfahren nach Anspruch 7, dadurch gekennzeichnet, daß als Alkohol Isopropanol verwendet wird.

9. Verfahren nach einem der vorangehenden Ansprüche, dadurch gekennzeichnet, daß wenigtens eine Überzugsmasse mit einem Gehalt an Stärke und/oder einem schwerlöslichen und/oder mit der eßbaren organischen Säure nicht raktionsfähigen Alkali- und/oder Erdalkalimetallsalz verwendet wird.

10. Verfahren nach einem der vorangehenden Ansprüche, dadurch gekennzeichnet, daß mindestens eine Vorreaktionslösung mit einem Gehalt an Stärke und/oder einem schwerlöslichen und/oder mit der eßbaren organischen Säure nicht reaktionsfähigen Alkali- und/oder Erdalkalimetallsalz verwendet wird.

11. Verfahren nach Anspruch 9 oder 10, dadurch gekennzeichnet, daß als nicht reaktionsfähiges Salz Calciumlactat verwendet wird.

12. Verfahren nach einem der Ansprüche 9 bis 10, dadurch gekennzeichnet, daß als nicht reaktionsfähiges Salz Calciumlaevulat verwendet wird.

13. Verfahren nach einem der Ansprüche 9 bis 10, dadurch gekennzeichnet, daß als nicht reaktionsfähiges Salz Calciumgluconat, vorzugsweise Calciumheptagluconat, verwendet wird.

14. Verfahren nach einem der Ansprüche 11 bis 13, dadurch gekennzeichnet, daß die (jeweilige) Überzugsschicht mit einem Gehalt an nicht raktionsfähigem Calciumsalz von, bezogen auf den jeweiligen Alkali- und/oder Erdalkalimetallcarbonatgehalt, 5 bis 30 Gew.-% hergestellt wird.

15. Verfahren nach einem der Ansprüche 9 bis 14, dadurch gekennzeichnet, daß die (jeweilige) stärkehaltige Überzugsschicht mit einem Gehalt an wasserfreier Stärke von, bezogen auf den jeweiligen Alkali- und/oder Erdalkalimetallcarbonatgehalt, 2 bis 8 Gew.-% hergestellt wird.

16. Brausegranulat, hergestellt nach einem der vorangehanden Ansprüche.

17. Verwendung des Brausegranulates nach Anspruch 16 als Instant — Granulat zum Herstellen von Brausegetränken oder dergleichen.

18. Verwendung des Brausegranulates nach Anspruch 16 zum Herstellen von Brausetabletten.

19. Verwendung nach Anspruch 17 oder 18, dadurch gekennzeichnet, daß das Brausegranulat bis zu einem Verhältnis von 1:5 mit Instantzucker gemischt wird.

**Revendications**

1. Procédé de préparation de granulés effervescents contenant au moins un acide organique solide cristallisé alimentaire et au moins un carbonate de métal alcalin ou alcalino-terreux libérant $CO_2$ à la réaction avec l'acide organique en solution aqueuse, caractérisé par les stades opératoires suivants:

(a) on dissout dans un solvant de l'acide et on fait réagir entre elles une partie de la quantité totale d'acide et une partie du ou des carbonates de métaux alcalins d'une première masse de revêtement en poudre contenant au moins un carbonate de métal alcalin ou alcalino-terreux;

(b) ou mouille par la solution de réaction préalable préparée au stade opératoire (a) les cristaux d'acide non utilisés au stade opératoire (a) pour la préparation de cette solution de réaction préalable;

(c) on soumet les cristaux d'acide mouillés par la solution de réaction préalable au stade opératoire (b) à mélange intensif avec la partie de la première masse de revêtement en poudre non utilisée pour la préparation de la solution de réaction préalable, ce qui conduit à la formation sur les cristaux d'acide d'une première couche de revêtement qui adhère à la surface des cristaux d'acide par une couche de liaison formée par réaction de l'acide et du carbonate;

(d) après formation de la première couche de revêtement, on ajoute sous agitation intensive aux cristaux d'acide sur lesquels on a appliqué une première couche de revêtement au stade opératoire (c) au moins une autre masse de revêtement solide en poudre contenant au moins un carbonate de métal alcalin ou alcalino-terreux, ce qui conduit à la formation d'au moins une autre couche de revêtement sur la couche de revêtement précédente humide de l'eau de cristallisation libérée;

(e) lorsque la formation du revêtement est terminée, on procède à un séchage final.

2. Procédé selon la revendication 1, caractérisé en ce que, après formation de la première couche de revêtement et/ou d'au moins une des couches de revêtement suivantes, on procède à un mouillage intermédiaire par une autre solution de réaction préalable formée dans chaque cas dans un complément de solvant par réaction préalable entre une autre partie (respective) de la quantité totale d'acide et une partie du ou des carbonates de métaux alcalins et/ou alcalino-terreux de la masse de revêtement à appliquer immédiatement à la suite.

3. Procédé selon la revendication 1 ou 2, caractérisé en ce que l'on réchauffe le solvant à une température d'environ 30 à 40 degrés C avant introduction de l'acide et/ou du ou des carbonates de métaux alcalins ou alcalino-terreux.

4. Procédé selon l'une des revendications qui précèdant, caractérisé en ce que l'on réchauffe à une température d'environ 30 à 40 degrés C la ou les solutions de réaction préalables avant application sur les cristaux d'acide.

5. Procédé selon l'une des revendications qui précèdent, caractérisé en ce que l'on prépare la ou les solutions de réaction préalables à une concentration telle qu'elles soient juste encore limpides et qu'il ne se produise pas de cristallisation visible dans les délais nécessaires pour l'exécution des opérations.

6. Procédé selon l'une des revendications qui précèdent, caractérisé en ce que la ou les masses de revêtement solides en poudre contiennent chacune une partie de la quantité totale d'acide.

7. Procédé selon l'une des revendications qui précèdent, caractérisé en ce que, après application d'au moins une couche de revêtement et le cas échéant de la dernière couche de revêtement, on procède à au moins une application d'un alcool, suivie d'un séchage.

8. Procédé selon la revendication 7, caractérisé en ce que l'alcool utilisé est l'isopropanol.

9. Procédé selon l'une des revendications qui précèdent, caractérisé en ce que l'on utilise au moins une masse de revêtement contenant de l'amidon et/ou un sel de métal alcalin et/ou alcalino-terreux peu soluble et/ou non réactif avec l'acide organique alimentaire.

10. Procédé selon l'une des revendications qui précèdent, caractérisé en ce que l'on utilise au moins une solution de réaction préalable contenant de l'amidon et/ou un sel de métal alcalin et/ou alcalino-terreux peu soluble et/ou non réactif avec l'acide organique alimentaire.

11. Procédé selon la revendication 9 ou 10, caractérisé en ce que l'on utilise en tant que sel non réactif le lactate de calcium.

12. Procédé selon l'une des revendications 9 à 10, caractérisé en ce que l'on utilise en tant que sel non réactif le lévulate de calcium.

13. Procédé selon l'une des revendications 9 à 10, caractérisé en ce que l'on utilise en tant que sel non réactif un gluconate de calcium et de préférence le glucoheptonate de calcium.

14. Procédé selon l'une des revendications 11 à 13, caractérisé en ce que l'on prépare chacune des couches de revêtement à une teneur en sel de calcium non réactif de 5 à 30% en poids par rapport à la teneur respective en carbonate de métal alcalin et/ou alcalino-terreux.

15. Procédé selon l'une des revendications 9 à 14, caractérisé en ce que l'on prépare chacune des couches de revêtement contenant de l'amidon à une teneur en amidon anhydre de 2 à 8% en poids par rapport à la teneur respective en carbonate de métal alcalin et/ou alcalino-terreux.

16. Granulés effervescents préparés selon l'une des revendications qui précèdent.

17. Utilisation des granulés effervescents selon la revendication 16 en tant que granulés instantanés pour la préparation de boissons effervescents ou de produits analogues.

18. Utilisation des granulés effervescents selon la revendication 16 pour la préparation de comprimés effervescents.

19. Utilisation selon la revendication 17 ou 18, caractérisée en ce que l'on mélange les granulés effervescents avec du sucre instanté à des proportions relatives allant jusqu'à 1:5.

## Claims

1. A method of producing an effervescent granulate containing at least one solid crystalline edible organic acid and at leat one alkali-metal or alkaline-earth metal carbonate which splits off $CO_2$ on reacting with the organic acid in aqueous solution, characterised by the following steps:

(a) A part of the total quantity of acid and a part of the alkali-metal carbonate or carbonates in a first pulverized coating substance containing at least one alkali-metal or alkaline-earth metal carbonate are dissolved in a solvent for the acid and reacted together;

(b) The acid crystals not used in step (a) for producing the pre-reaction solution formed therein are wetted with the pre-reaction solution produced in step (a);

(c) The acid crystals wetted with the pre-reaction solution in step (b) are intensively and thoroughly mixed with the part of the first pulverized coating substance not used for producing the pre-reaction solution, with the result that a first coating layer is formed on the acid crystals and is stuck to the surface thereof by a bonding layer formed by reaction between the acid and carbonate;

(d) After the first coating layer has formed, at least one additional solid pulverized coating substance containing at least one alkali-metal or alkaline-earth metal carbonate is added with vigorous agitation to the acid crystals provided with the first coating layer in step (c), thus forming at least one additional coating layer on the previous coating layer, which is wet with split-off water or crystallization;

(e) The coating process is stopped, followed by final drying.

2. A method according to claim 1, characterised in that after the first coating layer and/or at least one of the subsequent coating layers has formed, intermediate wetting is brought about with an additional prereaction solution formed by prereacting a (respective) additional part of the total amount of acid and a part of the alkali-metal and/or alkaline-earth metal carbonate of the next-following coating substance in an additional solvent.

3. A method according to claim 1 or 2, characterised in that the solvent is heated to about 30 to 40°C before introducing the acid and/or the alkali-metal or alkaline-earth metal carbonate or carbonates.

4. A method according to any of the preceding claims, characterised in that the pre-reaction solution or solutions are heated to about 30 to 40°C before applying to the acid crystals.

5. A method according to any of the preceding claims, characterised in that the pre-reaction solution or solutions are produced in a concentration such that they are still clear and no visible crystallization occurs in them during the time required for working the method.

6. A method according to any of the preceding claims, characterised in that the solid pulverulent coating substance or substances each contain a part of the total quantity of acid.

7. A method according to any of the preceding claims, characterised in that after at least one coating layer (the last if required) has been applied, alcohol is applied at least once followed by drying.

8. A method according to claim 7, characterised in that the alcohol used is isopropanol.

9. A method according to any of the preceding claims, characterised by use of at least one coating substance containing starch and/or an alkali-metal and/or alkaline-earth metal salt which is difficultly soluble and/or does not react with the edible organic acid.

10. A method according to any of the preceding claims, characterised by use of at least one pre-reaction solution containing starch and/or an alkali-metal and/or alakline-earth metal salt which is difficultly soluble and/or does not react with the edible organic acid.

11. A method according to claim 9 or 10, characterised in that the non-reactive salt used is calcium lactate.

12. A method according to claim 9 or 10, characterised in that the non-reactive salt used is calcium laevulate.

13. A method according to claim 9 or 10, characterised in that the non-reactive salt used is calcium gluconate, preferably calcium hepta-gluconate.

14. A method according to any of claims 11 to 13, characterised in that the or each coating layer is produced with a content of 5 to 30 wt.% of non-reactive calcium salt relative to the respective content of alkali-metal and/or alkaline-earth metal carbonate.

15. A method according to any of claims 9 to 14, characterised in that the or each starch-containing coating layer is manufactured with a content of 2 to 8 wt.% of anhydrous starch relative to the content of the respective alkali-metal and/or alkaline-earth metal carbonate.

16. An effervescent granulate produced according to any of the preceding claims.

17. Use of the effervescent granulate according to claim 16 as an instant granulate for producing effervescent drinks or the like.

18. Use of the effervescent granulate according to claim 16 for producing effervescent tablets.

19. Use according to claim 17 or 18, characterised in that the effervescent granulate is mixed with instant sugar in a proportion of up to 1:5.